# EUROPEAN PATENT SPECIFICATION

(11) **EP 4 686 453 B1**
(45) Date of publication and mention of the grant of the patent: **21.10.2026**
(21) Application number: 24192465.3
(22) Date of filing: 01.08.2024
(51) Int. Cl.: A61B 17/70

(54) **RECEIVING PART OF A BONE ANCHORING DEVICE AND SYSTEM OF AT LEAST ONE INSTRUMENT AND A RECEIVING PART**
AUFNAHMETEIL EINER KNOCHENVERANKERUNGSVORRICHTUNG UND SYSTEM AUS MINDESTENS EINEM INSTRUMENT UND EINEM AUFNAHMETEIL
PARTIE DE RÉCEPTION D'UN DISPOSITIF D'ANCRAGE OSSEUX ET SYSTÈME D'AU MOINS UN INSTRUMENT ET D'UNE PARTIE DE RÉCEPTION

(43) Date of publication of application: 04.02.2026
(73) Proprietor: Biedermann Technologies GmbH & Co. KG, 78166 Donaueschingen (DE)
(72) Inventor: BIEDERMANN, Timo, 78647 Trossingen (DE); BIEDERMANN, Lutz, 78048 VS-Villingen (DE)
(74) Representative: Prüfer & Partner mbB Patentanwälte · Rechtsanwälte

(56) References cited:
- EP-A1- 3 878 386
- EP-B1- 2 713 913
- US-B2- 11 490 931
- US-B2- 11 627 996

## Description

The invention relates to a receiving part that may be part of a coupling device for coupling a bone anchoring element to a stabilization element such as a rod. In particular, the receiving part may be equally configured for engagement with a first instrument and a second instrument different from the first instrument, in particular for use in spinal surgery. The invention further relates to a coupling device with such a receiving part and to system of at least one instrument and a receiving part.

A receiving part of a bone anchoring device is known from US 2016/0361093 A1, the receiving part including a substantially U-shaped recess that forms two free legs. A groove is provided in an outer surface of each of the legs, wherein in a plane that includes the central axis of the receiving part and that extends through a center of each leg, a contour of a surface of the groove has at least a first arcuate portion and a second arcuate portion opposing the first arcuate portion, and the contour is substantially free from straight portions.

Various other receiving parts are known with engagement structures for instruments, for example from WO 2018/191131 A1, WO 2023/022886 A2, EP 3 878 386 A1, EP 2 713 913 B1 and US 11,490,931 B2.

It is an object of the present invention to provide an improved receiving part for a bone anchoring device and a coupling device with such a receiving part that can in particular facilitate and/or support engagement of the receiving part with an instrument, and wherein at least two different instruments can be used selectively and interchangeably with the receiving part. Furthermore, it is an object to provide a system of such a receiving part and at least one instrument.

The object is solved by a receiving part according to claim 1, a coupling device according to claim 13 and a system of at least one instrument and a receiving part according to claim 14.

Further developments are given in the dependent claims.

According to an embodiment, a receiving part of a bone anchoring device is provided, the receiving part extending along a central axis from a first end to a second end, wherein a substantially U-shaped-recess is provided adjacent to the first end for receiving a rod, the substantially U-shaped recess forming two free legs of the receiving part. The receiving part comprises a circumferential groove in an outer surface of at least one of the legs, the groove being delimited in the direction of the central axis by an upper surface and a lower surface, and a recess in the outer surface of the at least one leg, the recess being delimited in the direction of the central axis by an upper surface and a lower surface. At the outer surface of the receiving part, the lower surface of the recess and the lower surface of the groove are provided at the same distance from the first end of the receiving part.

The groove and/or the recess may serve for engagement of an instrument with the receiving part. The upper and lower surfaces of the groove and recess can provide for safe attachment of the instrument with the receiving part and can in particular allow a force to be transmitted from the instrument onto the receiving part, while preventing inadvertent detachment of the instrument from the receiving part.

By providing the lower surfaces of both the recess and the groove at the same axial height of the receiving part at its outer surface, a reproducible position of an instrument attached to the receiving part may be provided, even when using different instruments. Moreover, by providing both lower surfaces at the same axial height at the outer surface, it can be ensured that when using an instrument engaging only one of the groove and the recess, a coupling process of attaching the instrument to the receiving part is not interfered by the other one of the groove and the recess.

The receiving part can be manufactured in an easy and cost-efficient manner. Moreover, the receiving part may be used with different instruments that differ with respect to their engagement structures for engaging the receiving part. Specifically, a first instrument may be configured for engagement with the groove of the receiving part and a second instrument may be configured for engagement with the recess of the receiving part. The first instrument may be configured to only engage the groove and not the recess, whereas the second instrument may be configured to only engage the recess and not the groove.

Hence, the receiving part may be combined with existing instruments of different types, which can increase the versatility of the receiving part. This may reduce the costs of the inventory necessary for surgery.

A bone anchoring device comprising the receiving part and a bone anchoring element may be configured in any suitable form. For example, the bone anchoring device can be a polyaxial bone anchoring device or a monoplanar bone anchoring device or a monoaxial bone anchoring device. The bone anchoring device may be of the bottom-loading type in which a head of the bone anchoring device is insertable from the bottom end into the receiving part or of the top-loading type in which the head is insertable from the top end into the receiving part.

According to a further embodiment, a coupling device is provided for coupling a bone anchoring element to a rod, the bone anchoring element comprising a shank for anchoring in bone, wherein the coupling device comprises the receiving part according to the above embodiment. Preferably, the bone anchoring element comprises a head and the receiving part is configured to receive the head of the bone anchoring element, wherein further preferably the coupling device comprises a locking device configured to cooperate with the receiving part to lock the head in the receiving part.

According to a further embodiment, a system of at least one instrument and a receiving part of the above embodiment are provided, wherein the instrument comprises a tubular portion having two arms, wherein at least one arm comprises at its inner surface at least one protrusion configured to engage the groove and/or the recess of the receiving part. Preferably, the instrument is a first instrument and the at least one protrusion is formed as a circumferentially extending rib configured to engage the groove of the receiving part, and/or the instrument is a second instrument and the at least one protrusion is formed in a middle portion of the arm with respect to a circumferential direction of the second instrument and configured to engage the recess of the receiving part.

The receiving part, the coupling device and the system each may be use in particular in spinal surgery and more specifically in minimally invasive surgery (MIS).

Further features and advantages of the invention will become apparent from the description of embodiments by means of the accompanying drawings. In the drawings:
- Fig. 1: shows a perspective view of a bone anchoring device comprising a receiving part according to an embodiment.
- Fig. 2: shows a perspective view of the receiving part of Fig. 1.
- Fig. 3: shows a perspective view of the receiving part depicted in Figs. 1 and 2 from slightly above.
- Fig. 4: shows a perspective view of the receiving part depicted in Figs. 1 to 3 from slightly below.
- Fig. 5: shows an enlarged view of a portion of the receiving part as depicted in Fig. 4.
- Fig. 6: shows an enlarged view of a portion of the receiving part as depicted in Fig. 3.
- Fig. 7: shows an enlarged view of another portion of the receiving part depicted in Fig. 3.
- Fig. 8: shows a side view of the receiving part depicted in Figs. 1 to 7.
- Fig. 9: shows another side view of the receiving part depicted in Figs. 1 to 7.
- Fig. 10: shows an enlarged cross-sectional view of a detail of the receiving part as depicted in Fig. 9 in a cross-section taken along line A-A in Fig. 9.
- Fig. 11: shows an enlarged cross-sectional view of a detail of the receiving part as depicted in Fig. 9 in a cross-section taken along line B-B in Fig. 9.
- Fig. 12: shows a perspective view of a portion of an instrument according to a first embodiment.
- Fig. 13: shows a perspective view of the bone anchoring device of Fig. 1 with a portion of the instrument of Fig. 12 attached thereto.
- Fig. 14: shows a cross-sectional view of the bone anchoring device with a portion of the instrument as depicted in Fig. 13, the cross-section taken in a plane perpendicular to a rod axis of an inserted rod.
- Fig. 15: shows a perspective view of a portion of an instrument according to a second embodiment.
- Fig. 16: shows a perspective view of the bone anchoring device of Fig. 1 with a portion of the instrument of Fig. 15 attached thereto.
- Fig. 17: shows a cross-sectional view of the bone anchoring device with a portion of the instrument as depicted in Fig. 16, the cross-section taken in a plane perpendicular to a rod axis of an inserted rod.
- Fig. 18: shows a side view of an example of another receiving part.
- Fig. 19: shows a side view of an example of yet another receiving part.

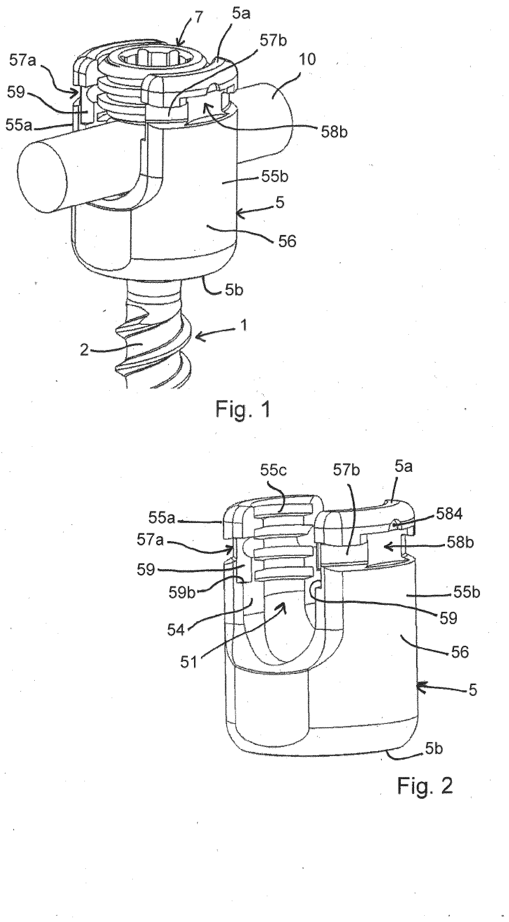

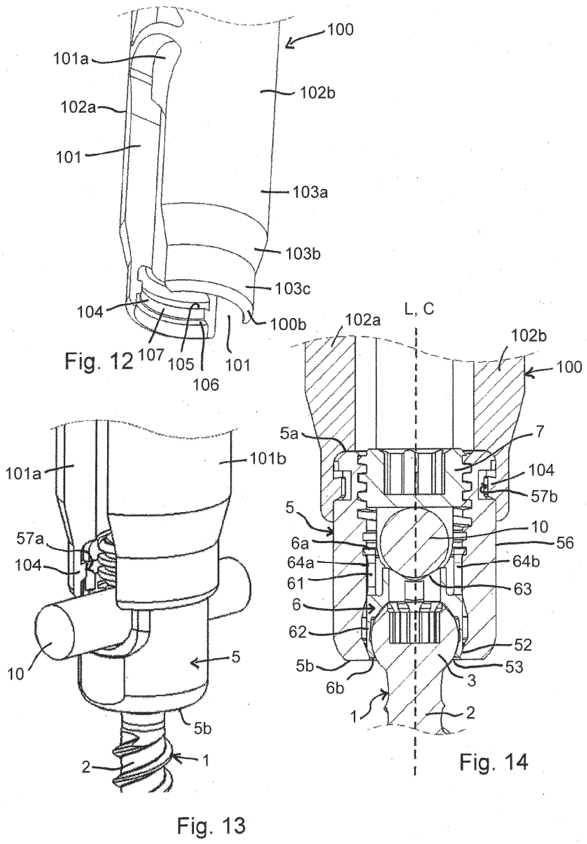

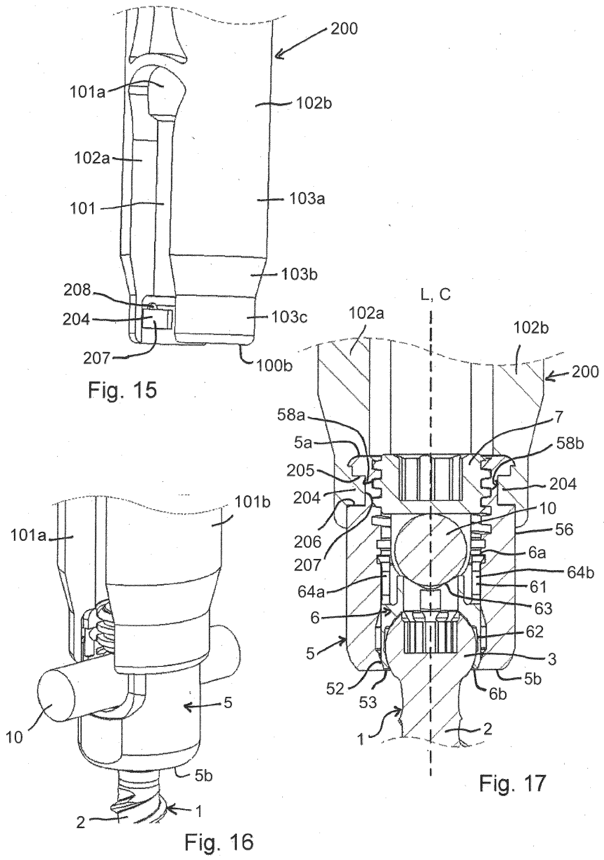

As shown in Fig. 1, and with additional reference to Figs. 14 and 17, a bone anchoring device according to an embodiment includes a bone anchoring element 1 with a threaded shank 2 and a head 3 with a spherical outer surface portion. The bone anchoring device further comprises a receiving part 5 for coupling the head 3 of the bone anchoring element 1 to a spinal fixation element, for example a rod 10. A pressure element 6 is provided at least partially within the receiving part 5, the pressure element 6 being configured to exert pressure onto the inserted head 3 to lock the head 3 in the receiving part 5. A fixation member, for example a set screw 7, is provided for fixing the rod 10 in the receiving part 5. The pressure element 6 and the head 3 are shown in Figs. 14 and 17.

The receiving part 5 is shown in more detail in Figs. 2 to 11. The receiving part 5 may be a monolithic part. Typically, the receiving part 5 may be formed as a substantially cylindrical part with a first end or top end 5a, a second end or bottom end 5b and a central axis C extending through the top end 5a and the bottom end 5b. A passage 51 extends from the top end 5a to the bottom end 5b along the central axis C. Close to the bottom end 5b of the receiving part 5, a narrowing portion 52 in the passage 51 provides for a seat for the head 3 of the anchoring element 1. At the bottom end 5b of the receiving part 5, a lower opening 53 is formed that permits the shank 2 to extend therethrough. The receiving part 5 further comprises a substantially U-shaped recess 54 that extends from the top end 5a in the direction of the bottom end 5b. The U-shaped recess 54 serves for receiving the rod 10 therein. Two free legs 55a, 55b of the receiving part 5 are formed by the U-shaped recess 54. **In** an upper portion of the legs 55a, 55b, an internal thread 55c may be provided for cooperating with the set screw 7 for fixing the rod 10. The receiving part 5 may have rounded edges at its top end 5a.

**In** an outer surface 56 of each of the legs 55a, 55b a circumferential groove 57a, 57b and a recess 58a, 58b are formed, respectively. The circumferential grooves 57a, 57b and/or the recesses 58a, 58b may serve as engagement portions for engagement of an instrument with the receiving part 5, as explained in more detail below. More generally, the recesses 58a, 58b each form a notch that intersects the corresponding groove 57a, 57b.

The circumferential grooves 57a, 57b and the recesses 58a, 58b each extend from the outer surface 56 of the receiving part 5 towards the central axis C. Thus, the thickness of the legs 55a, 55b in the radial direction may be reduced by the grooves 57a, 57b and by the recesses 58a, 58b. At each of the legs 55a, 55b, the circumferential groove 57a, 57b and the respective recess 58a, 58b intersect each other in the center of the leg in the circumferential direction.

The circumferential groove 57a, 57b of each leg 55a, 55b is provided at a distance from the top end 5a of the receiving part 5. Moreover, the circumferential grooves 57a, 57b extend from one end of the channel formed by the U-shaped recess 54 to the opposite end of the channel and are open towards the U-shaped recess 54. In other words, the circumferential grooves 57a, 57b extend in a circumferential direction of the receiving part 5, i.e. in a plane perpendicular to the central axis C, in an arcuate shape along the entire circumferential extension of the respective leg 55a, 55b.

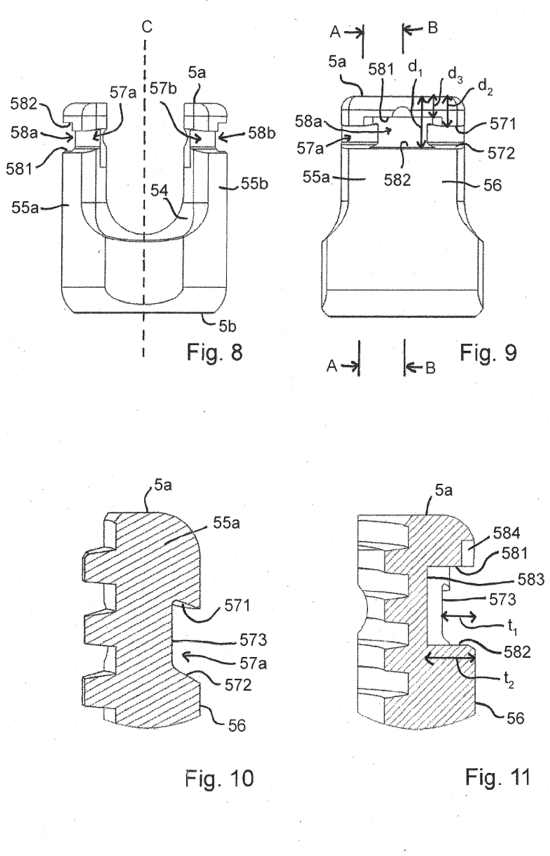

Each of the circumferential grooves 57a, 57b is delimited towards the upper end 5a of the receiving part 5 by an upper surface 571 of the groove, and is delimited towards the bottom end 5b by a lower surface 572 of the groove. As can best be seen in Fig. 9, at the outer surface 56 of the receiving part 5, the lower surface 572 of the groove 57a, 57b is provided at a first distance d₁ from the top end 5a of the receiving part 5 and the upper surface 571 is provided at a second distance d₂ from the top end 5a of the receiving part 5, the second distance d₂ being smaller than the first distance d₁.

The upper surface 571 and the lower surface 572 of the groove 57a, 57b may be inclined from the outer surface 56 of the receiving part towards the top end 5a. In the present embodiment, the upper surface 571 and the lower surface 572 are substantially planar faces that extend from the outer surface 56 into the receiving part 5 in a depth direction thereof. A sidewall 573 or bottom of the groove 57a, 57b delimits the groove in the depth direction. The sidewall 573 may be planar and may connect to the upper and lower surfaces 571, 572 at rounded edges.

It shall be understood that the upper and lower surfaces 571, 572, as well as the sidewall 573, are not limited to planar faces. Rather, they can have any other shape, such as a rounded or arcuate shape. Depending on the shape of the upper and lower surfaces 571, 572, the sidewall 573 may also be omitted.

The groove 57a, 57b has a depth t₁ extending from the outer surface 56 of the receiving part 5 to a point of the groove extending furthest inwards into the receiving part. In the present embodiment, the groove 57a, 57b has the depth t₁ between the outer surface 56 and the sidewall 573, as depicted in Figs. 10 and 11.

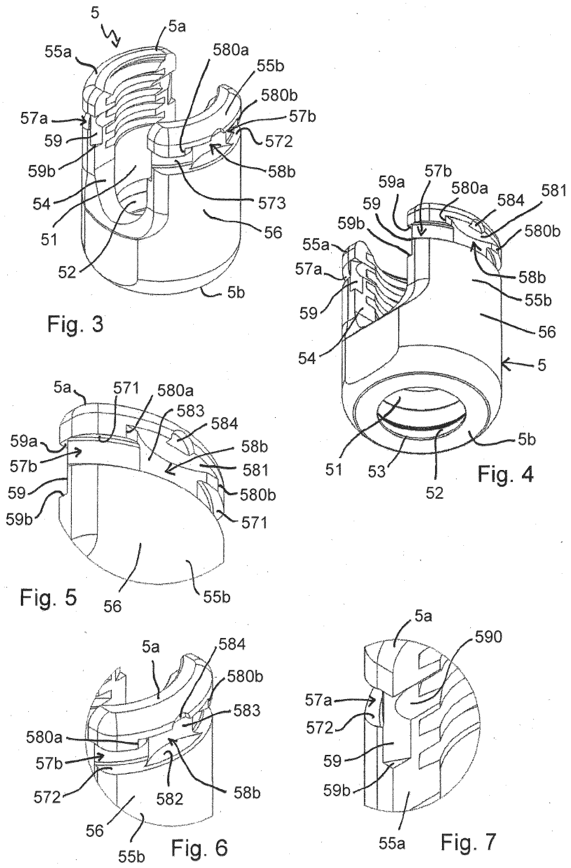

The recess 58a, 58b of each leg 55a, 55b is provided in the middle of the respective leg in the circumferential direction. In the axial direction, the recess 58a, 58b is located at a distance from the top end 5a of the receiving part. In the circumferential direction of the respective leg 55a, 55b, the recess 58a, 58b extends from a first end 580a to a second end 580b of the recess as can be seen in particular in Figs. 4 to 6. A width of the recess 58a, 58b between the first end 580a and the second end 580b is smaller than the circumferential extension of the groove 57a, 57b. In the outer surface 56 of the receiving part, a contour of the recess 58a, 58b may have a substantially rectangular shape.

Each of the recesses 58a, 58b is delimited towards the upper end 5a of the receiving part 5 by an upper surface 581, and is delimited towards the bottom end 5b by a lower surface 582. As can best be seen in Fig. 9, at the outer surface 56 of the receiving part 5, the lower surface 582 of the recess 58a, 58b is provided at the same first distance d₁ from the top end 5a of the receiving part 5 as the lower surface 572 of the respective groove 57a, 57b. In particular, the lower surface 582 of the recess 58a, 58b may be flush with the lower surface 572 of the groove 57a, 57b.

The upper surface 581 of the recess 58a, 58b is provided at a third distance d₃ from the top end 5a of the receiving part 5, wherein the third distance d₃ may be smaller than the second distance d₂ of the upper surface 571 of the groove 57a, 57b from the top end 5a of the receiving part 5. By means of the upper surface 581 and the lower surface 582, the recess 58a, 58b is closed towards both the top end 5a and the bottom end 5b of the receiving part. The upper surface 581 and the lower surface 582 of the recess 58a, 58b may extend substantially perpendicular to the central axis C in a direction from the outer surface 56 of the receiving part towards the central axis C, i.e., towards the passage 51. In the present embodiment, the upper surface 581 and the lower surface 582 are substantially planar faces that extend from the outer surface 56 into the receiving part 5 in a depth direction thereof. A sidewall 583 or bottom of the recess 58a, 58b delimits the recess in the depth direction.

The recess 58a, 58b has a maximum depth t₂ from the outer surface 56 of the receiving part 5 to a point of the recess extending furthest inwards into the receiving part 5. The sidewall 583 of the recess 58a, 58b may have a curved shape that extends from each end 580a, 580b at the outer surface 56 of the receiving part 5 inwards into the receiving part. Preferably, the shape of the sidewall 583 is cylindrical with the cylinder axis being substantially parallel to the central axis C.

As can be seen from Fig. 11, the maximum depth t₂ of the recess 58a, 58b exceeds the depth t₁ of the groove 57a, 57b.

It shall be understood that the upper and lower surfaces 581, 582 and the sidewall 583 of the recess 58a, 58b are not limited in their shape to the planar faces and curved shape of the present embodiment. Rather, they can have any other shape, for example, the upper and lower surfaces can have a rounded or arcuate shape, and/or the sidewall 583 can have a planar shape. Depending on the shape of the upper and lower surfaces 581, 582, the sidewall 583 may also be omitted, e.g., if the upper and lower surfaces are inclined towards one another or have a rounded or arcuate shape so that they meet at their positions furthest away from the outer surface 56 (not shown in the figures).

The recess 58a, 58b may further comprise a notch 584 provided in the outer surface 56 of the receiving part that extends from the upper surface 582 of the recess 58a, 58b towards the upper end 5a of the receiving part. The notch 584 may be located in the middle of the recess 58a, 58b between its two ends 580a, 580b, and may have a curved shape. A depth of the notch 584 may be smaller than the depth of the recess 58a, 58b and the depth of the groove 57a, 57b.

Preferably, the groove 57a and recesses 58a provided on a first leg 55a of the receiving part are arranged and formed to be symmetrical with respect to the groove 57b and recess 58b of the second leg 58b with regard to a plane that includes the central axis C and passes centrally through the U-shaped recess 54 of the receiving part 5.

In addition, the legs 55a, 55b may comprise cutouts 59 at each circumferential end of each leg 55a, 55b that are facing the substantially U-shaped recess 54. Each cutout 59 may extend in axial direction from a first end 59a of the cutout 59 located at the upper surface 571 of the groove 57a, 57b to a second end 59b of the cutout 59 located closer to the bottom end 5b of the receiving part 5 than the lower surface 572 of the groove 57a, 57b.

In addition, a bevelled surface 590 may be provided that is located below the upper surface of each groove 57a, 57b and extends from each cutout 59 towards the internal thread 55c. The bevelled surface 590 may facilitate insertion of a portion of an instrument into the inside of the receiving part 5.

The pressure element 6 that can best be seen in the cross-sectional view of Figs. 14 and 17 may comprise a substantially cylindrical first portion 61 at an upper end 6a of the pressure element 6a and a substantially cylindrical second portion 62 at a lower end 6b thereof. The substantially cylindrical second portion 62 may be configured to extend at least partially around an inserted head 3 and may be at least partially flexible to clamp an inserted head. For example, the second portion 62 may comprise a plurality of longitudinal slits (not shown in the figures) that render the second portion 62 flexible. Clamping of an inserted head 3 may be achieved by moving the pressure member 6 downwards within the passage 51 of the receiving part 5 so that the second portion 62 of the pressure member 6 is compressed by the narrowing portion 52 of the receiving part 5.

The first portion 61 of the pressure element 6 may comprise a base 63 having a substantially V-shaped or U-shaped contour, which base 63 forms a rod support surface for an inserted rod, and two upstanding legs 64a, 64b at both sides of the base 63. An upper end of the legs 64a, 64b may provide for a stop that cooperates with the receiving part to prevent upward movement of the pressure element 6 within the receiving part 5.

A coupling device according to an embodiment, which serves for coupling a bone anchoring element 1 to a rod 10, may comprise the receiving part 5 for receiving the rod 10 and the head 3 of the bone anchoring element 1 therein and a locking device in the form of the pressure element 6 that is configured to exert pressure onto the head 3 to lock the head 3 in the receiving part 5. The coupling device together with the bone anchoring element may, for example, form a polyaxial bone anchoring device where the shank can pivot relative to the receiving part, and/or a monoplanar bone anchoring device where the pivoting motion of the shank relative to the receiving part is restricted to a single plane, and/or a monoaxial bone anchoring device where the shank can assume a single predetermined angle with respect to the receiving part. The bone anchoring device may be of the bottom-loading type in which the head is insertable from the bottom end 5b into the receiving part or of the top-loading type in which the head is insertable from the top end 5a into the receiving part.

The receiving part can have other shapes as shown in the embodiment. In particular the receiving part can have any shape that permits to receive the head of a bone anchoring device in a pivotable manner. Also, the receiving part can have a structure which allows to pivot the bone anchoring element to one side at a greater angle than to another side. For a monoaxial coupling device the receiving part can be fixedly connected to the shank or can be monolithically formed with a shank thereby omitting a head.

The pressure element can have other shapes as shown in the embodiment. The pressure element 6 of the embodiment described above is a monolithic part. However, the pressure element may be formed of two or more parts that are configured to cooperate with one another to lock the head in the receiving part. The pressure element can also only cover and inserted head from above, such that the head can pivot in a seat provided directly in the receiving part. Instead of a pressure element, any other locking device configured to lock the head in the receiving part can be implemented, such as an outer ring at least partially arranged around a head receiving portion of the receiving part and configured to exert a clamping force onto the head receiving portion to lock an inserted head therein.

Parts and portions of the receiving part and the coupling device may be made of any material, preferably, however, of a bio-compatible material, such as titanium or stainless steel or any other bio-compatible metal or metal alloy, or plastic material. As bio-compatible alloy, a Ni-Ti-alloy, for example Nitinol, may be used. Other materials can be Magnesium or Magnesium alloys, bio-compatible plastic materials for use may be for example, Polyether ether ketone (PEEK) or Poly-L-lactide acid (PLLA). The parts can be made of the same or of different mate-rials from one another.

Next, an instrument according to a first embodiment, which is configured to engage the receiving part 5, will be described with additional reference to Figs. 12 to 14. This embodiment is an example of an instrument configured to engage the grooves 57a, 57b. In the figures, only a lower part of a tubular portion 100 of the instrument is shown. Specifically, the tubular portion 100 of the instrument extends from an upper end (not shown in the figures) to a second end 100b along a longitudinal axis L. The tubular portion 100 may be substantially cylindrical in shape.

Two longitudinal slits 101 are provided in the tubular portion 100 that are open towards the second end 100b and extend from the second end 100b in the direction of the longitudinal axis L. Each slit may terminate in an enlarged portion 101a at a distance from the second end 100b. By means of the slits 101, two arms 102a, 102b of the tubular portion 100 are formed. The slits 101 render the tubular portion at least partially flexible, so that the arms 102a, 102b are expandable away from each other and compressible towards each other.

An outer diameter of the tubular portion 100 may decrease towards the second end 100b. For example, in the present embodiment, the tubular portion 100 of the instrument comprises a first cylinder-segment shaped portion 103a and a second cylinder-segment shaped portion 103c adjacent the second end 100b, wherein an outer diameter of the second cylinder-segment shaped portion 103c is smaller than a diameter of the first cylinder-segment shaped portion 103a. The outer diameter may gradually decrease form the first to the second cylinder-segment shaped portion to form an intermediate narrowing portion 103b.

An inner diameter of the tubular portion 100 is such that the arms smoothly fit around the outer surface 56 of the receiving part to engage the receiving part there between.

At an inner surface of the second cylinder-segment shaped portion 103c a circumferentially extending rib 104 is provided at each arm 102a, 102b which is configured to engage the circumferential groove 57a, 57b provided at the outer surface 56 of the arms 55a, 55b of the receiving part 5.

Specifically, the circumferential rib 104 may comprise an upper surface 105 and a lower surface 106 delimiting the rib towards the first end (not shown) and the second end 100b of the tubular portion 100, respectively, and a sidewall 107 connecting the upper and lower surfaces 105, 106 of the rib. The upper and lower surfaces 105, 106 may be inclined with respect to the longitudinal axis L and are formed such that the rib 104 fits into the groove 57a, 57b, respectively. In greater detail, the upper and lower surfaces 105, 106 abut on the upper and lower surfaces 571, 572 of the groove 57a, 57b, respectively, and the sidewall 107 of the circumferential rib 104 may abut on the sidewall 573 of the groove 57a, 57b when the tubular portion 100 is attached to the receiving part 5.

The circumferential rib 104 may extend along an entire width of the respective arm 102a, 102b in the circumferential direction thereof.

In use, the tubular portion 100 of the instrument is advanced with its second end 100b towards the upper end 5a of the receiving part 5 to place the tubular portion onto the receiving part. Further advancement of the tubular portion 100 towards the bottom end 5b of the receiving part spreads the arms 102a, 102b of the tubular portion apart. The rounded edges of the receiving part 5a at its top end may facilitate and assist in spreading the arms of the tubular portion 100. Finally, the circumferential ribs 104 provided at the inner surfaces of the arms 102a, 102b snap into the circumferential grooves 57a, 57b of the respective leg 55a, 55b of the receiving part 5, which causes the arms 102a, 102b to compress again to thus couple the tubular portion 100 to the receiving part 5.

Alternatively, for attaching the tubular portion 100 to the receiving part 5, the tubular portion 100 can be advanced towards the receiving part with its arms 102a, 102b located at the position of the substantially U-shaped-recess 54 of the receiving part with regard to the circumferential direction, i.e., circumferentially between the legs 55a, 55b of the receiving part 5. When the circumferential ribs 104 are located at the position of the circumferential grooves 57a, 57b in the axial direction, the tube 100 is rotated around its longitudinal axis L so that the circumferential ribs 104 enter the circumferential grooves 57a, 57b.

A second embodiment of an instrument configured to engage the receiving part 5 will be described with additional reference to Figs. 15 to 17. This embodiment is an example of an instrument configured to engage the recesses 58a, 58b In the figures, only a lower part of a tubular portion 200 of the instrument is shown. The tubular portion 200 of the second instrument is similar to the tubular portion 100 of the first instrument described above, and in the figures similar features and elements are denoted by the same reference numbers. In the following, only the differences of the second instrument to the first instrument will therefore be described.

Instead of the circumferential rib 104 of the tubular portion 100 of the first instrument, the tubular portion 200 of the second instrument includes at an inner surface of the second cylinder-segment shaped portion 103c a protrusion 204 provided at each arm 102a, 102b that is configured to engage the recesses 58a, 58b provided at the outer surface 56 of the receiving part 5.

The protrusion 204 may be formed in a middle portion of the respective arm 102a, 102b with respect to a circumferential direction thereof. In the circumferential direction of the arms, the protrusion 204 may extend along a length that is equal to or only slightly smaller than the width of the recess 58a, 58b of the receiving part 5 between the first end 580a and the second end 580b. A contour of the protrusion 204 in a plane of the inner surface of the arms may have a substantially rectangular shape.

The protrusion 204 may comprise an upper surface 205 and a lower surface 206 delimiting the protrusion towards the first end (not shown) and the second end 100b of the tubular portion 200, respectively, and a sidewall 207 connecting the upper and lower surfaces 205, 206 of the protrusion 204. The upper and lower surfaces 205, 206 may be substantially planar and extend substantially perpendicular to the longitudinal axis L. The sidewall 207 may have a curved shape that is complementary to the curved shape of the sidewall 583 of the recess 58a, 58b provided at the receiving part 5. A thickness of the protrusion 204 between the inner surface of the respective arm 102a, 102b and the sidewall 207 may be substantially equal to the depth of the recess 58a, 58b. More generally, the shape of the protrusion 204 may be such that the protrusion can engage the recess 58a, 58b. In greater detail, the upper and lower surfaces 205, 206 of the protrusion 204 may be formed and arranged such that they abut on the upper and lower surfaces 581, 582 of the recess 58a, 58b, respectively, and the sidewall 207 of the protrusion 204 may abut on the sidewall 583 of the recess 58a, 58b when the tubular portion 200 is attached to the receiving part 5.. However, the protrusion may not be configured to engage the grooves 57a, 57b.

The protrusion 204 may comprise a projection 208 that is shaped and arranged to engage the notch 584 of the recess 58a, 58b provided in the outer surface 56 of the receiving part 5 when the protrusion 204 engages the recess 58a, 58b.

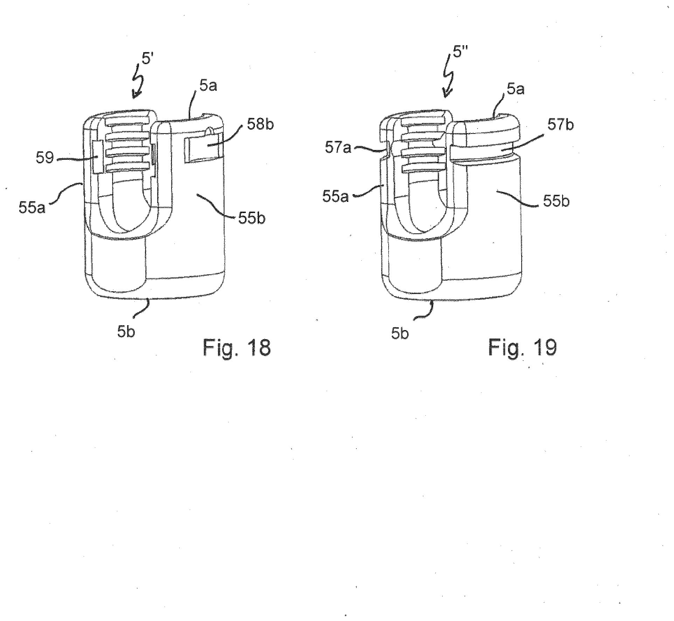

In an example of another receiving part 5' shown in Fig. 18, the receiving part is provided without the circumferential grooves 57a, 57b at the legs 55a, 55b. Hence, the receiving part 5' shown in Fig. 18 only comprises the recesses 58a, 58b provided at its legs 55a, 55b, and the receiving part 5' may be used together with the second instrument described above with reference to Figs. 15 to 17.

In an example of yet another receiving part 5" shown in Fig. 19, the receiving part is provided without the recesses 58a, 58b at the legs 55a, 55b. Hence, the receiving part 5" shown in Fig. 19 only comprises the circumferential grooves 57a, 57b provided at its legs 55a, 55b, and the receiving part 5" may be used together with the first instrument described above with reference to Figs. 12 to 14.

Thus, the receiving part 5 according to the invention as shown in Figs. 1 to 17 can be used with both instrument types one of which is configured to engage a receiving part 5' as shown in Fig. 18 and another which is configured to engage a receiving part 5" as shown in Fig. 19.

## Claims

1. A receiving part of a bone anchoring device, the receiving part (5) comprising
a first end (5a), a second end (5b) opposite to the first end (5a), a central axis (C) extending through the first end (5a) and the second end (5b),
a substantially U-shaped-recess (54) adjacent to the first end (5a) for receiving a rod (10), wherein the substantially U-shaped recess (54) forms two free legs (55a, 55b),
a circumferential groove (57a, 57b) in an outer surface (56) of at least one of the legs (55a, 55b), the groove (57a, 57b) being delimited in the direction of the central axis (C) by an upper surface (571) and a lower surface (572), and
a recess (58a, 58b) in the outer surface (56) of the at least one leg, the recess (58a, 58b) being delimited in the direction of the central axis (C) by an upper surface (581) and a lower surface (582), wherein the recess (58a, 58b) is closed towards both the first end (5a) and the second end (5b) of the receiving part, and
wherein at the outer surface (56) of the receiving part (5), the lower surface (582) of the recess (58a, 58b) and the lower surface (572) of the groove (57a, 57b) are provided at the same distance (d₁) from the first end (5a) of the receiving part (5).

2. The receiving part of claim 1, wherein at the outer surface (56) of the receiving part, the upper surface (581) of the recess (58a, 58b) is provided at a smaller distance from the first end (5a) of the receiving part than the upper surface (571) of the groove.

3. The receiving part of claim 1 or 2, wherein a depth (t₁) of the recess (58a, 58b) from the outer surface (56) of the receiving part towards the central axis (C) exceeds a depth (t₂) of the groove (57a, 57b) from the outer surface (56) of the receiving part towards the central axis (C).

4. The receiving part of one of claims 1 to 3, wherein the groove (57a, 57b) extends in a circumferential direction of the receiving part (5) in an arcuate shape along the entire circumferential extension of the at least one leg (55a, 55b).

5. The receiving part of one of claims 1 to 4, wherein the recess (58a, 58b) extends in a circumferential direction of the receiving part (5) from a first end (580a) to a second end (580b) along a length that is smaller than the circumferential extension of the groove (57a, 57b).

6. The receiving part of one of claims 1 to 5, wherein the recess (58a, 58b) extends in a circumferential direction of the receiving part from a first end (580a) to a second end (580b) and wherein a depth of the recess from the outer surface (56) of the receiving part towards the central axis (C) increases from the first and/or second end of the recess towards a middle portion of the recess spaced apart from the first end and the second end.

7. The receiving part of one of claims 1 to 6, wherein the groove (57a, 57b) intersects the recess (58a, 58b) in the circumferential direction.

8. The receiving part of one of claims 1 to 7, wherein the upper surface (571) and/or the lower surface (572) of the groove is inclined from the outer surface (56) of the receiving part towards the first end (5a).

9. The receiving part of one of claims 1 to 8, wherein the upper surface (581) and/or the lower surface (582) of the recess (58a, 58b) extends substantially perpendicular to the central axis (C).

10. The receiving part of one of claims 1 to 9, wherein a notch (584) is provided at the outer surface (56) of the at least one leg (55a, 55b), the notch (584) extending from the upper surface (581) of the recess (58a, 58b) towards the first end (5a) of the receiving part.

11. The receiving part of one of claims 1 to 10, wherein the at least one leg (55a, 55b) comprises a cutout (59) at the circumferential end of the leg that faces the substantially U-shaped recess (54), wherein the cutout (59) extends in the direction of the central axis (C) from a first end (59a) at the upper surface (571) of the groove (57a, 57b) to a second end (59b) of the cutout (59) beyond the lower surface (572) of the groove.

12. The receiving part of one of claims 1 to 11, wherein the groove (57a, 57b) and/or the recess (58a, 58b) is provided at both free legs (55a, 55b).

13. A coupling device for coupling a bone anchoring element (1) to a rod (10), the bone anchoring element (1) comprising a shank (2) for anchoring in bone, wherein the coupling device comprises
the receiving part (5) of one of claims 1 to 12,
wherein preferably the bone anchoring element comprises a head and the receiving part being configured to receive the head (3) of the bone anchoring element, and
wherein further preferably the coupling device comprises a locking device (6) configured to cooperate with the receiving part (5) to lock the head (3) in the receiving part.

14. A system of at least one instrument and a receiving part of one of claims 1 to 12 or a coupling device of claim 13, wherein the instrument comprises a tubular portion (100, 200) having two arms (102a, 102b), wherein at least one arm comprises at its inner surface at least one protrusion (104, 204) configured to engage the groove (57a, 57b) and/or the recess (58a, 58b) of the receiving part (5),
wherein preferably the instrument is a first instrument and the at least one protrusion is formed as a circumferentially extending rib (104) configured to engage the groove (57a, 57b) of the receiving part, and/or
wherein preferably the instrument is a second instrument and the at least one protrusion (204) is formed in a middle portion of the arm with respect to a circumferential direction of the second instrument and configured to engage the recess (58a, 58b) of the receiving part.

## Patentansprüche

1. Aufnahmeteil einer Knochenverankerungsvorrichtung, wobei das Aufnahmeteil (5) umfasst:
ein erstes Ende (5a), ein zweites Ende (5b) gegenüber dem ersten Ende (5a), eine Mittelachse (C), die sich durch das erste Ende (5a) und das zweite Ende (5b) erstreckt,
eine im Wesentlichen U-förmige Ausnehmung (54) benachbart zu dem ersten Ende (5a) zum Aufnehmen eines Stabs (10), wobei die im Wesentlichen U-förmige Ausnehmung (54) zwei freie Schenkel (55a, 55b) bildet,
eine Umfangsnut (57a, 57b) in einer Außenfläche (56) von zumindest einem der Schenkel (55a, 55b), wobei die Nut (57a, 57b) in der Richtung der Mittelachse (C) durch eine obere Fläche (571) und eine untere Fläche (572) begrenzt ist, und
eine Ausnehmung (58a, 58b) in der Außenfläche (56) des zumindest einen Schenkels, wobei die Ausnehmung (58a, 58b) in der Richtung der Mittelachse (C) durch eine obere Fläche (581) und eine untere Fläche (582) begrenzt ist, wobei die Ausnehmung (58a, 58b) sowohl zu dem ersten Ende (5a) als auch zu dem zweiten Ende (5b) des Aufnahmeteils hin geschlossen ist, und
wobei an der Außenfläche (56) des Aufnahmeteils (5) die untere Fläche (582) der Ausnehmung (58a, 58b) und die untere Fläche (572) der Nut (57a, 57b) in dem gleichen Abstand (d₁) von dem ersten Ende (5a) des Aufnahmeteils (5) vorgesehen sind.

2. Aufnahmeteil nach Anspruch 1, wobei an der Außenfläche (56) des Aufnahmeteils die obere Fläche (581) der Ausnehmung (58a, 58b) in einem kleineren Abstand von dem ersten Ende (5a) des Aufnahmeteils vorgesehen ist als die obere Fläche (571) der Nut .

3. Aufnahmeteil nach Anspruch 1 oder 2, wobei eine Tiefe (t₁) der Ausnehmung (58a, 58b) von der Außenfläche (56) des Aufnahmeteils zu der Mittelachse (C) hin größer ist als eine Tiefe (t₂) der Nut (57a, 57b) von der Außenfläche (56) des Aufnahmeteils zu der Mittelachse (C) hin.

4. Aufnahmeteil nach einem der Ansprüche 1 bis 3, wobei sich die Nut (57a, 57b) in einer Umfangsrichtung des Aufnahmeteils (5) in einer bogenförmigen Form entlang der gesamten Umfangserstreckung des zumindest einen Schenkels (55a, 55b) erstreckt.

5. Aufnahmeteil nach einem der Ansprüche 1 bis 4, wobei sich die Ausnehmung (58a, 58b) in einer Umfangsrichtung des Aufnahmeteils (5) von einem ersten Ende (580a) zu einem zweiten Ende (580b) entlang einer Länge erstreckt, die kleiner ist als die Umfangserstreckung der Nut (57a, 57b).

6. Aufnahmeteil nach einem der Ansprüche 1 bis 5, wobei sich die Ausnehmung (58a, 58b) in einer Umfangsrichtung des Aufnahmeteils von einem ersten Ende (580a) zu einem zweiten Ende (580b) erstreckt und wobei eine Tiefe der Ausnehmung von der Außenfläche (56) des Aufnahmeteils zu der Mittelachse (C) hin von dem ersten und/oder dem zweiten Ende der Ausnehmung zu einem mittleren Abschnitt der Ausnehmung hin zunimmt, der von dem ersten Ende und dem zweiten Ende beabstandet ist.

7. Aufnahmeteil nach einem der Ansprüche 1 bis 6, wobei die Nut (57a, 57b) die Ausnehmung (58a, 58b) in der Umfangsrichtung schneidet.

8. Aufnahmeteil nach einem der Ansprüche 1 bis 7, wobei die obere Fläche (571) und/oder die untere Fläche (572) der Nut von der Außenfläche (56) des Aufnahmeteils zu dem ersten Ende (5a) hin geneigt ist.

9. Aufnahmeteil nach einem der Ansprüche 1 bis 8, wobei sich die obere Fläche (581) und/oder die untere Fläche (582) der Ausnehmung (58a, 58b) im Wesentlichen senkrecht zu der Mittelachse (C) erstreckt.

10. Aufnahmeteil nach einem der Ansprüche 1 bis 9, wobei eine Kerbe (584) an der Außenfläche (56) des zumindest einen Schenkels (55a, 55b) vorgesehen ist, wobei sich die Kerbe (584) von der oberen Fläche (581) der Ausnehmung (58a, 58b) in Richtung des ersten Endes (5a) des Aufnahmeteils erstreckt.

11. Aufnahmeteil nach einem der Ansprüche 1 bis 10, wobei der zumindest eine Schenkel (55a, 55b) einen Ausschnitt (59) an dem Umfangsende des Schenkels umfasst, der der im Wesentlichen U-förmigen Ausnehmung (54) zugewandt ist, wobei sich der Ausschnitt (59) in der Richtung der Mittelachse (C) von einem ersten Ende (59a) an der oberen Fläche (571) der Nut (57a, 57b) zu einem zweiten Ende (59b) des Ausschnitts (59) über die untere Fläche (572) der Nut hinaus erstreckt.

12. Aufnahmeteil nach einem der Ansprüche 1 bis 11, wobei die Nut (57a, 57b) und/oder die Ausnehmung (58a, 58b) an beiden freien Schenkeln (55a, 55b) vorgesehen ist.

13. Kopplungsvorrichtung zum Koppeln eines Knochenverankerungselements (1) mit einem Stab (10), wobei das Knochenverankerungselement (1) einen Schaft (2) zum Verankern in Knochen umfasst, wobei die Kopplungsvorrichtung umfasst:
das Aufnahmeteil (5) nach einem der Ansprüche 1 bis 12,
wobei vorzugsweise das Knochenverankerungselement einen Kopf umfasst und das Aufnahmeteil dazu ausgebildet ist, den Kopf (3) des Knochenverankerungselements aufzunehmen, und
wobei weiter bevorzugt die Kopplungsvorrichtung eine Verriegelungsvorrichtung (6) umfasst, die dazu ausgebildet ist, mit dem Aufnahmeteil (5) zusammenzuwirken, um den Kopf (3) in dem Aufnahmeteil zu verriegeln.

14. System aus mindestens einem Instrument und einem Aufnahmeteil nach einem der Ansprüche 1 bis 12 oder einer Kopplungsvorrichtung nach Anspruch 13, wobei das Instrument einen röhrenförmigen Abschnitt (100, 200) mit zwei Armen (102a, 102b) umfasst, wobei mindestens ein Arm an seiner Innenfläche mindestens einen Vorsprung (104, 204) umfasst, der dazu ausgebildet ist, mit der Nut (57a, 57b) und/oder der Ausnehmung (58a, 58b) des Aufnahmeteils (5) in Eingriff zu sein,
wobei vorzugsweise das Instrument ein erstes Instrument ist und der mindestens eine Vorsprung als eine sich in Umfangsrichtung erstreckende Rippe (104) ausgebildet ist, die dazu ausgebildet ist, mit der Nut (57a, 57b) des Aufnahmeteils in Eingriff zu sein, und/oder
wobei vorzugsweise das Instrument ein zweites Instrument ist und der mindestens eine Vorsprung (204) in einem mittleren Abschnitt des Arms in Bezug auf eine Umfangsrichtung des zweiten Instruments ausgebildet ist und dazu ausgebildet ist, mit der Ausnehmung (58a, 58b) des Aufnahmeteils in Eingriff zu sein.

## Revendications

1. Partie de réception d'un dispositif d'ancrage osseux, la partie de réception (5) comprenant
une première extrémité (5a), une seconde extrémité (5b) opposée à la première extrémité (5a), un axe central (C) s'étendant à travers la première extrémité (5a) et la seconde extrémité (5b),
un évidement sensiblement en forme de U (54) adjacent à la première extrémité (5a) pour recevoir une tige (10), dans laquelle l'évidement sensiblement en forme de U (54) forme deux branches libres (55a, 55b),
une rainure circonférentielle (57a, 57b) dans une surface extérieure (56) d'au moins l'une des branches (55a, 55b), la rainure (57a, 57b) étant délimitée dans la direction de l'axe central (C) par une surface supérieure (571) et une surface inférieure (572), et
un évidement (58a, 58b) dans la surface extérieure (56) de l'au moins une branche, l'évidement (58a, 58b) étant délimité dans la direction de l'axe central (C) par une surface supérieure (581) et une surface inférieure (582), dans lequel l'évidement (58a, 58b) est fermé à la fois vers la première extrémité (5a) et la seconde extrémité (5b) de la partie de réception, et
dans lequel au niveau de la surface extérieure (56) de la partie de réception (5), la surface inférieure (582) de l'évidement (58a, 58b) et la surface inférieure (572) de la rainure (57a, 57b) sont prévues à la même distance (d₁) de la première extrémité (5a) de la partie de réception (5).

2. Partie de réception selon la revendication 1, dans laquelle au niveau de la surface extérieure (56) de la partie de réception, la surface supérieure (581) de l'évidement (58a, 58b) est prévue à une distance plus petite de la première extrémité (5a) de la partie de réception que la surface supérieure (571) de la rainure.

3. Partie de réception selon la revendication 1 ou 2, dans laquelle une profondeur (t₁) de l'évidement (58a, 58b) de la surface extérieure (56) de la partie de réception vers l'axe central (C) dépasse une profondeur (t₂) de la rainure (57a, 57b) de la surface extérieure (56) de la partie de réception vers l'axe central (C).

4. Partie de réception selon l'une des revendications 1 à 3, dans laquelle la rainure (57a, 57b) s'étend dans une direction circonférentielle de la partie de réception (5) selon une forme arquée le long de toute l'extension circonférentielle de l'au moins une branche (55a, 55b).

5. Partie de réception selon l'une des revendications 1 à 4, dans laquelle l'évidement (58a, 58b) s'étend dans une direction circonférentielle de la partie de réception (5) d'une première extrémité (580a) à une seconde extrémité (580b) le long d'une longueur qui est plus petite que l'extension circonférentielle de la rainure (57a, 57b).

6. Partie de réception selon l'une des revendications 1 à 5, dans laquelle l'évidement (58a, 58b) s'étend dans une direction circonférentielle de la partie de réception d'une première extrémité (580a) à une seconde extrémité (580b) et dans laquelle une profondeur de l'évidement de la surface extérieure (56) de la partie de réception vers l'axe central (C) augmente de la première et/ou de la seconde extrémité de l'évidement vers une partie centrale de l'évidement espacée de la première extrémité et de la seconde extrémité.

7. Partie de réception selon l'une des revendications 1 à 6, dans laquelle la rainure (57a, 57b) coupe l'évidement (58a, 58b) dans la direction circonférentielle.

8. Partie de réception selon l'une des revendications 1 à 7, dans laquelle la surface supérieure (571) et/ou la surface inférieure (572) de la rainure est inclinée de la surface extérieure (56) de la partie de réception vers la première extrémité (5a).

9. Partie de réception selon l'une des revendications 1 à 8, dans laquelle la surface supérieure (581) et/ou la surface inférieure (582) de l'évidement (58a, 58b) s'étend sensiblement perpendiculairement à l'axe central (C).

10. Partie de réception selon l'une des revendications 1 à 9, dans laquelle une encoche (584) est prévue au niveau de la surface extérieure (56) de l'au moins une branche (55a, 55b), l'encoche (584) s'étendant de la surface supérieure (581) de l'évidement (58a, 58b) vers la première extrémité (5a) de la partie de réception.

11. Partie de réception selon l'une des revendications 1 à 10, dans laquelle l'au moins une branche (55a, 55b) comprend une découpe (59) au niveau de l'extrémité circonférentielle de la branche qui fait face à l'évidement sensiblement en forme de U (54), dans laquelle la découpe (59) s'étend dans la direction de l'axe central (C) d'une première extrémité (59a) au niveau de la surface supérieure (571) de la rainure (57a, 57b) à une seconde extrémité (59b) de la découpe (59) au-delà de la surface inférieure (572) de la rainure.

12. Partie de réception selon l'une des revendications 1 à 11, dans laquelle la rainure (57a, 57b) et/ou l'évidement (58a, 58b) est prévu au niveau des deux branches libres (55a, 55b).

13. Dispositif de couplage pour coupler un élément d'ancrage osseux (1) à une tige (10), l'élément d'ancrage osseux (1) comprenant un manche (2) pour l'ancrage dans l'os, dans lequel le dispositif de couplage comprend
la partie de réception (5) selon l'une des revendications 1 à 12,
dans lequel de préférence l'élément d'ancrage osseux comprend une tête et la partie de réception étant configurée pour recevoir la tête (3) de l'élément d'ancrage osseux, et
dans lequel en outre de préférence le dispositif de couplage comprend un dispositif de verrouillage (6) configuré pour coopérer avec la partie de réception (5) pour verrouiller la tête (3) dans la partie de réception.

14. Système d'au moins un instrument et d'une partie de réception selon l'une des revendications 1 à 12 ou d'un dispositif de couplage selon la revendication 13, dans lequel l'instrument comprend une partie tubulaire (100, 200) ayant deux bras (102a, 102b), dans lequel au moins un bras comprend au niveau de sa surface intérieure au moins une saillie (104, 204) configurée pour venir en prise avec la rainure (57a, 57b) et/ou l'évidement (58a, 58b) de la partie de réception (5),
dans lequel de préférence l'instrument est un premier instrument et l'au moins une saillie est formée comme une nervure s'étendant circonférentiellement (104) configurée pour venir en prise avec la rainure (57a, 57b) de la partie de réception, et/ou
dans lequel de préférence l'instrument est un second instrument et l'au moins une saillie (204) est formée dans une partie centrale du bras par rapport à une direction circonférentielle du second instrument et configurée pour venir en prise avec l'évidement (58a, 58b) de la partie de réception.
